# EUROPÄISCHE PATENTSCHRIFT

(11) **EP 1 802 311 B1**
(45) Veröffentlichungstag und Bekanntmachung des Hinweises auf die Patenterteilung: **18.03.2009**
(21) Anmeldenummer: 05805172.3
(22) Anmeldetag: 01.10.2005
(51) Int. Cl.: A61K 31/5377, C07D 413/12, A61P 31/12

(54) **4-AMINOPHENYL-MORPHOLINON-DERIVATE ZUR BEHANDLUNG VON HEPATITIS C**
4-AMINOPHENYL MORPHOLINONE DERIVATIVES FOR THE TREATMENT OF HEPATITIS C
DERIVES DE 4-AMINOPHENYL-MORPHOLINONE POUR TRAITER L'HEPATITE C

(30) Priorität: 15.10.2004 DE 102004050283
(43) Veröffentlichungstag der Anmeldung: 04.07.2007
(73) Patentinhaber: Saltigo GmbH, 40764 Langenfeld (DE)
(72) Erfinder: MARHOLD, Albrecht, 51373 Leverkusen (DE); EBENBECK, Wolfgang, 51373 Leverkusen (DE)
(74) Vertreter: Pettrich, Klaus-Günter
(86) Internationale Anmeldenummer: PCT/EP2005/010625
(87) Internationale Veröffentlichungsnummer: WO 2006/042634

(56) Entgegenhaltungen:
- WO-A-20/04033450
- WO-A-20/04087695
- DATABASE WPI Section Ch, Week 200422 Derwent Publications Ltd., London, GB; Class B03, AN 2004-235586 XP002362397 & KR 2003 079 396 A (BNC BIO PHARM CO LTD) 10. Oktober 2003 (2003-10-10)

## Beschreibung

Die Erfindung betrifft neue Aminophenyl-morpholinon-Derivate, ein Verfahren zu deren Herstellung sowie deren Verwendung. 4-(Morpholin-4-yl)-anilin oder 4-(3-Oxo-morpholin-4-yl)-anilin sind wichtige Bausteine für die Herstellung pharmazeutischer Wirkstoffe.

Aus WO-A 2004/033450 ist beispielsweise bekannt, dass die Verbindung 6-Methyl-2-[4-(4-morpholino)-anilin]-nicotinsäure aufgrund ihres inhibierenden Effekts bei der Replikation des Hepatitis C Virus als pharmazeutischer Wirkstoff zur Behandlung von Hepatitis C geeignet ist. Jedoch weist die Verbindung hinsichtlich Wirkhöhe, Löslichkeit für eine geeignete Darreichungsform sowie in der Pharmakokinese Nachteile auf.

Es bestand daher weiterhin der Bedarf nach Wirkstoffen, die die vorangehenden Nachteile nicht aufweisen und sich zur therapeutischen Behandlung beispielsweise von Erkrankungen durch Viren, insbesondere von Hepatitis C, eignen.

Der vorliegenden Erfindung lag somit die Aufgabe zugrunde, solche Verbindungen bereitzustellen.

Überraschend wurde nun gefunden, dass Pyrimidin- und Pyridincarbonsäurederivate, die in 2- bzw. 4-Position durch einen gegebenenfalls substituierten 4-(3-Oxo-morpholin-4-yl)-anilin-Rest substituiert sind, diese Anforderungen erfüllen.

Gegenstand der vorliegenden Erfindung sind daher Verbindungen der allgemeinen Formel (I), 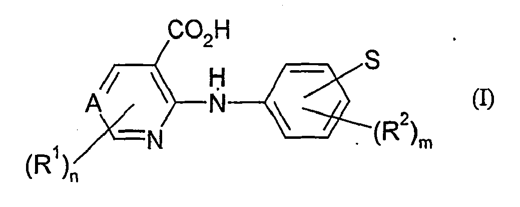 worin
- A: für CH, CR¹ oder N, bevorzugt für CH steht,
- R¹: unabhängig voneinander für H, OH, C₁-C₄-Alkyl, teil- oder perfluoriertes C₁-C₄-Alkyl, C₁-C₄-Alkoxy, teil- oder perfluoriertes C₁-C₄-Alkoxy, Amino, Mono- oder Diamino-C₁-C₄-alkyl, Nitro, Carboxyl, C(S)NH₂, C(O)NH₂, Cyano oder Halogen steht,
- n: für 0, 1 oder 2, bevorzugt für 0 oder 1 steht,
- R²: unabhängig voneinander für H, OH, C₁-C₄-Alkyl, teil- oder perfluoriertes C₁-C₄-Alkyl, C₁-C₄-Alkoxy, teil- oder perfluoriertes C₁-C₄-Alkoxy, Amino, Mono- oder Diamino-C₁-C₄-alkyl, Nitro, Carboxyl, C(S)NH₂, C(O)NH₂, Cyano oder Halogen steht,
- m: für 0, 1, 2, 3 oder 4, bevorzugt für 0 oder 1 steht,
- S: für einen Rest ausgewählt aus den allgemeinen Formeln (II-a) bis (II-d) 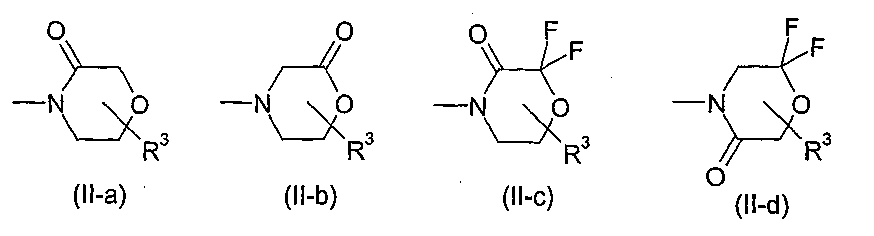 steht, worin
- R³: für H, C₁-C₄-Alkyl, gegebenenfalls substituiertes C₄-C₂₄-Aryl, bevorzugt gegebenenfalls substituiertes C₆-C₂₄-Aryl, oder gegebenenfalls substituiertes C₅-C₁₈-Aralkyl steht,
oder Salze der Verbindungen der allgemeinen Formel (I) oder Solvate oder Hydrate der Verbindungen der allgemeinen Formel (I) oder deren Salzen.

Salze der erfindungsgemäßen Verbindungen können physiologisch unbedenkliche Salze der erfindungsgemäßen Verbindungen der allgemeinen Formel (I) mit anorganischen oder organischen Säuren sein. Bevorzugt werden Salze mit anorganischen Säuren wie beispielsweise Chlorwasserstoffsäure, Bromwasserstoffsäure, Phosphorsäure oder Schwefelsäure, oder Salze mit organischen Carbon- oder Sulfonsäuren wie beispielsweise Essigsäure, Trifluoressigsäure, Propionsäure, Maleinsäure, Fumarsäure, Äpfelsäure, Zitronensäure, Weinsäure, Milchsäure, Benzoesäure oder Methansulfonsäure, Ethansulfonsäure, Benzolsulfonsäure, Toluolsulfonsäure oder Naphthalinsulfonsäure.

Salze der erfindungsgemäßen Verbindungen können ebenso physiologisch unbedenkliche Salze der erfindungsgemäßen Verbindungen der allgemeinen Formel (I) mit üblichen Basen sein, wie beispielsweise Alkalimetallsalze, wie z.B. Natrium- oder Kaliumsalze, Erdalkalimetallsalze, wie z.B. Calcium- oder Magnesiumsalze, Ammoniumsalze, abgeleitet von Ammoniak oder organischen Aminen, wie beispielsweise Diethylamin, Triethylamin, Ethyldiispropylamin, Prokain, Dibenzylamin, N-Methylmorpholin, Dihydroabietylamin oder Methylpiperidin, oder Salze von Ethanoklarminen, wie beispielsweise 2-Diethylaminoethanol, 2-[(2-Hydroxy-ethyl)methyl-amino]-ethanol.

Als Hydrate oder Solvate werden erfindungsgemäß solche Formen der erfindungsgemäßen Verbindungen der allgemeinen Formel (I) bezeichnet, die in festem oder flüssigem Zustand durch Hydratation mit Wasser oder Koordination mit Lösungsmittelmolekülen eine Molekül-Verbindung oder einen Komplex bilden. Beispiele für Hydrate sind Sesquihydrate, Monohydrate, Dihydrate oder Trihydrate. Gleichermaßen kommen auch Hydrate oder Solvate von Salzen der erfindungsgemäßen Verbindungen der allgemeinen Formel (I) in Betracht.

**Alkyl** oder **Alkoxy** bedeutet jeweils unabhängig einen linearen, verzweigten oder unverzweigten Alkyl- oder Alkylen-Rest Gleiches gilt für den nichtaromatischen Teil eines Arylalkyl-Restes sowie für Alkylbestandteile komplexerer Substituenten wie z.B. Mono- oder Diaminalkyl.

**C₁-C₄-Alkyl** steht beispielsweise für Methyl, Ethyl, n-Propyl, iso-Propyl, n-Butyl, sec.-Butyl, tert.-Butyl.

**C₁-C₄-Alkoxy** steht beispielsweise für Methoxy, Ethoxy, n-Propoxy, iso-Propoxy, n-Butoxy, sec.-Butoxy, tert.-Butoxy.

**Aryl** steht jeweils unabgängig für einen aromatischen Rest mit 4 bis 24 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, ersetzt sein können, vorzugsweise jedoch für einen carbocyclischen aromatischen Rest mit 6 bis 24 Gerüstkohlenstoffatomen.

Beispiele für **C₆-C₂₄-Aryl** sind Phenyl, o-, p-, m-Tolyl, Naphthyl, Phenanthrenyl, Anthracenyl oder Fluorenyl, Beispiele für heteroaromatisches **C₄-C₂₄-Aryl** in denen keines, ein, zwei oder drei Gerüstkohlenstofftome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, sind beispielsweise Pyridyl, Pyridyl-N-Oxid, Pyrimidyl, Pyridazinyl, Pyrazinyl, Thienyl, Furyl, Pyrrolyl, Pyrazolyl, Imidazolyl, Thiazolyl, Oxazolyl oder Isoxazolyl, Indolizinyl, Indolyl, Benzo[b]thienyl, Benzo[b]furyl, Indazolyl, Chinolyl, Isochinolyl, Naphthyridinyl, Chinazolinyl, Benzofuranyl oder Dibenzofuranyl.

**Arylalkyl** bedeutet jeweils unabhängig einen geradkettigen, cyclischem, verzweigten oder unverzweigten Alkyl-Rest nach obiger Definition, der einfach, mehrfach oder vollständig durch Aryl-Reste gemäß obiger Definition substituiert sein kann.

C₅-C₁₈-Arylalkyl steht beispielsweise für Benzyl oder (R)- oder (S)-1-Phenylethyl.

Als mögliche Substituenten für die Reste R³ kommen zahlreiche organische Gruppen in Frage, beispielsweise Alkyl-, Cycloalkyl-, Aryl-, Halogen-, Hydroxyl-, Ether-, Thioether-, Disulfid-, Sulfoxid-, Sulfonsäure-, Sulfonat-, Amino-, Aldehyd-, Keto-, Carbonsäureester-, Carbonat-, Carboxylat, Cyano-, Alkylsilan- und Alkoxysilangruppen sowie Carboxylamidgruppen.

Bevorzugt steht R¹ für H, F, OH, Methyl, Methoxy, Trifluormethyl oder Difluormethyl, besonders bevorzugt für H, F oder Methyl.

R² steht bevorzugt für H, F, Cl, CN, OH, Methyl, Methoxy, Trifluormethyl, Difluormethyl, C(S)NH₂ oder C(O)NH₂, besonders bevorzugt für H, F oder Methyl.

R³ steht bevorzugt für H, C₁-C₄-Alkyl, gegebenenfalls substituiertes Phenyl oder Benzyl, besonders bevorzugt für H oder Methyl.

Bevorzugt befindet sich S in p-Stellung zum Aminosubstituenten des gegebenenfalls substituierten Aminophenylringes in der allgemeinen Formel (I).

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

In bevorzugten Ausführungsformen der Erfindung stehen R¹ und R² unabhängig voneinander für H, F oder Methyl und S für einen Rest der allgemeinen Formel (II-a), 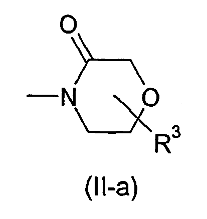 worin R³ für H oder Methyl steht.

Beispielhaft seien hier die Verbindungen der Formeln (I-a) bis (I-c) genannt.

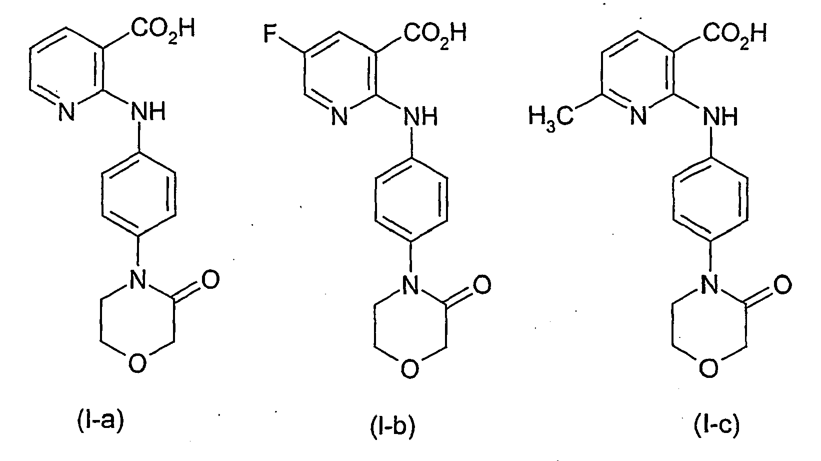

Für den Fall, dass die erfindungsgemäßen Verbindungen der allgemeinen Formel (I) ein Chiralitätszentrum aufweisen, sind unter erfindungsgemäßen Verbindungen der allgemeinen Formel (I) im Rahmen der Erfindung die einzelnen Enantiomeren dieser Verbindungen in reiner oder angereicherter Form, die Racemate oder Mischungen der einzelnen Enantiomeren in beliebigem Verhältnis umfasst.

Weiterhin Gegenstand der vorliegenden Erfindung ist ein Verfahren zur Herstellung der erfindungsgemäßen Verbindungen der allgemeinen Formel (I), wobei Verbindungen der allgemeinen Formel (III), 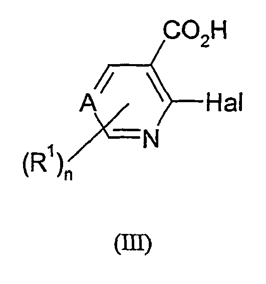 worin
- Hal: für Cl, Br oder I, bevorzugt für Cl oder Br, besonders bevorzugt für Cl steht und
- A und R¹: die für die allgemeine Formel (I) angegebene Bedeutung haben,
mit Verbindungen der allgemeinen Formel (IV) 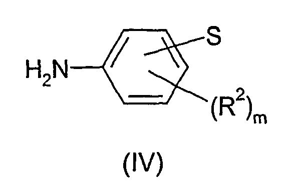 worin R² und S die für die allgemeine Formel (I) angegebene Bedeutung haben,
umgesetzt werden.

Das erfindungsgemäße Verfahren wird gegebenenfalls in Gegenwart organischer Basen wie beispielsweise aliphatischer oder aromatischer Amine oder Diamine durchgeführt. Bevorzugt sind aromatische Amine wie beispielsweise Pyridin, 2-Methyl-, 3-Methyl-, 4-Methyl-, 2,4-Dimethyl-, 3,4-Dimethyl-, 3,5-Dimethyl- oder 2,6-Dimethylpyridin, 5-Ethyl-2-methyl-pyridin, 2-, 3- oder 4-N,N-Dimethylamino-pyridin, N,N-Dimethyl-anilin, N,N-Dimethyl-benzylamin oder N-Methylpiperidin, aliphatische Amine wie beispielsweise Trimethylamin, Triethylamin, Tri-isopropylamin, Tri-n-propylamin, Tri-butyl-amin, Di-isopropyl-ethylamin, N,N-Dimethyl-cyclohexylamin, Dicyclohexylamin, Dicyclohexyl-ethylamin, Tri-n-octylamin, Di-n-octyl-methylamin, Diamine wie beispielsweise 1,3-Dimethylimidazolidin oder Diazabicycloalkane, wie z.B. 1,4-Di-aza-bicyclo-[2.2.2]-octan (DABCO), 1,5-Di-aza-bicyclo-[4.3.0]-non-5-en (DBN), oder 1,8-Di-aza-bicyclo-[5.4.0]-undec-7-en (DBU).

Das erfindungsgemäße Verfahren wird bevorzugt in einem oder mehreren Lösungsmittel(n) durchgeführt. Geeignete Lösungsmittel sind beispielsweise organische Lösungsmittel wie Alkohole, z.B. Methanol, Ethanol, n-Propanol, Isopropylalkohol, n-, sec- oder tert-Butanol oder Amylalkohol, Ketone, z.B. Aceton, Ether, z.B. Diethylether, Methyl-tert-butylether, Dioxan oder Tetrahydrofuran, aliphatische oder aromatische gegebenenfalls chlorierte Kohlenwasserstoffe, z.B. Dichlormethan, Chloroform, 1,2-Dichlorethan oder Toluol, Xylol oder Carbonsäurederivate, z.B. Acetonitril oder N,N-Dimethylformamid, N,N-Dimethylacetamid, N-Methylpyrrolidon (NMP) oder Dimethylimidazolidinon (DMI), Sulfone oder Sulfoxide, wie z.B. Dimethylsulfoxid (DMSO) oder Mischungen aus zwei oder mehreren dieser Lösungsmittel. Besonders bevorzugt sind Methanol, Ethanol, Isopropylalkohol, Dichlormethan, Chloroform, Aceton, Acetonitril, N,N-Dimethylformamid, N,N-Dimethylacetamid, N-Methylpyrrolidon (NMP), Dimethylimidazolidinon (DMI), Dimethylsulfoxid (DMSO).

Das erfindungsgemäße Verfahren wird bevorzugt bei Temperaturen von 40°C bis 150°C, besonders bevorzugt 40°C bis 110°C, ganz besonders bevorzugt unter Rückfluss bei der Siedetemperatur des verwendeten Lösungsmittels oder Lösungsmittelgemisches durchgeführt. Die Reaktionszeit beträgt bevorzugt mehrere Stunden bis Tage, besonders bevorzugt einen bis 15 Tage, ganz besonders bevorzugt einen bis 10 Tage.

Die Durchführung des erfindungsgemäßen Verfahrens unter Schutzgasatmosphäre, wie z.B. Stickstoff- oder Argonatmosphäre, kann von Vorteil sein, ist jedoch nicht zwingend erforderlich.

Das erfindungsgemäße Verfahren kann bei normalem, erhöhtem oder vermindertem Druck durchgeführt werden, beispielsweise im Bereich von 0,5 bis 5 bar. Im Allgemeinen wird es bei Normaldruck durchgeführt.

Das erfindungsgemäße Verfahren wird beispielsweise so durchgeführt, dass die Verbindung der allgemeinen Formel (III), die Verbindung der allgemeinen Formel (IV) sowie die organische Base in dem oder den entsprechenden Lösungsmittel(n) für die entsprechende Reaktionszeit mittels Rühren unter Rückfluss umgesetzt werden. Nach Abkühlen, beispielsweise auf Temperaturen von 10°C bis 30°C, bevorzugt Raumtemperatur, kann die Verbindung der allgemeinen Formel (I) nach Kristallisation und Filtration isoliert werden. Gegebenenfalls kann mittels Entfernen des Lösungsmittels aus dem Filtrat und Aufnahme der Rückstandes in weiterem oder weiteren Lösungsmittel(n) und erneutem vollständigen oder teilweisem Entfernen des oder der Lösungsmittel(s) und gegebenenfalls nachfolgender Kristallisation die Ausbeute weiter erhöht werden. Als weitere Lösungsmittel sind die vorangehend genannten geeignet, wobei diese unabhängig vom oder von den für die Umsetzung verwendeten Lösungsmittel(n) gleich oder verschieden von diesen sein können. Bevorzugt werden für die Isolierung andere Lösungsmittel eingesetzt als für die Umsetzung.

Die Verbindungen der allgemeinen Formel (III) sind kommerziell erhältlich. Die Verbindungen der allgemeinen Formel (IV) können beispielsweise nach bekannten Verfahren, wie beispielsweise in WO-A 01/47919 oder WO-A 02/064575 beschrieben, hergestellt werden.

Die im erfindungsgemäßen Verfahren eingesetzten Verbindungen der allgemeinen Formel (IV), 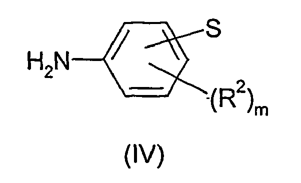 worin R² die für die Verbindungen der allgemeinen Formel (I) angegebene Bedeutung hat und S für einen Rest ausgewählt aus den allgemeinen Formeln (II-b) bis (II-d) 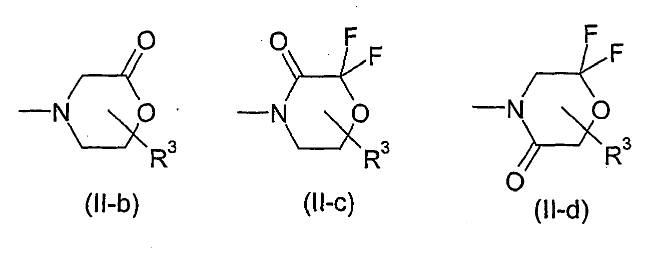 steht,
worin R³ die für die Verbindungen der allgemeinen Formel (I) angegebene Bedeutung hat, sind ebenfalls neu und somit Gegenstand der vorliegenden Verbindung.

Beispielhaft seien hier die Verbindungen der Formel (IV-c) oder (IV-d) 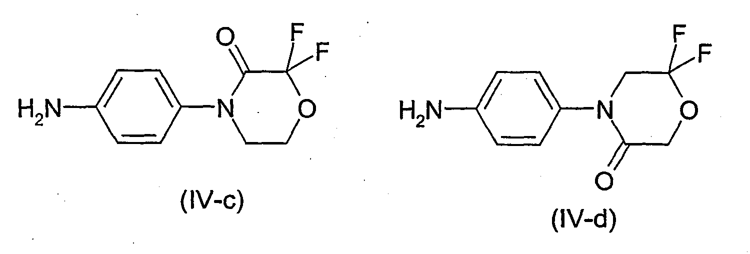 aufgeführt.

Diese erfindungsgemäßen Verbindungen der allgemeinen Formel (IV) eignen sich zur Herstellung pharmazeutischer Wirkstoffe, der allegemeinen Formel (I) sowohl gemäß dem erfindungsgemäßen Verfahren als auch gemäß anderer Verfahren. Ihre Verwendung ist somit nicht auf den Einsatz im erfindungsgemäßen Verfahren beschränkt.

Die erfindungsgemäßen Verbindungen der allgemeinen Formel (I) bzw. deren Salze, Solvate und/oder Hydrate eignen sich zur Verwendung als pharmazeutische Wirkstoffe. Die erfindungsgemäßen Verbindungen der allgemeinen Formel (I) bzw. deren Salze, Solvate und/oder Hydrate zeigen ein nicht vorhersehbares, wertvolles pharmakologisches Wirkspektrum und sind daher zur Behandlung von Erkrankungen geeignet. Insbesondere wirken die erfindungsgemäßen Verbindungen der allgemeinen Formel (I) bzw. deren Salze, Solvate und/oder Hydrate antiviral. Besonders bevorzugt können sie zur Behandlung von Hepatitis C eingesetzt werden.

Für die Applikation der erfindungsgemäßen Verbindungen der allgemeinen Formel (I) bzw. deren Salze, Solvate und/oder Hydrate kommen alle üblichen Applikationsformen in Betracht, wie beispielsweise oral, lingual, sublingual, bukkal, rektal oder parenteral, d.h. unter Umgehung des Intestinaltraktes, also intravenös, intraarteriell, intrakardial, intrakutan, subkutan, transdermal, intraperitoneal oder intramuskulär.

Die erfindungsgemäßen Verbindungen der allgemeinen Formel (I) bzw. deren Salze, Solvate und/oder Hydrate können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei sollte die therapeutisch wirksame erfindungsgemäße Verbindung bevorzugt in einer Konzentration von 0,1 bis 95 Gew.-%, besonders bevorzugt in 0,5 bis 90 Gew.-% der Gesamtmischung vorhanden sein, d.h. in Mengen, die ausreichend sind, um den geeigneten Dosierungsspielraum zu erreichen.

Trotzdem kann es erforderlich sein, von den zuvor genannten Mengen abzuweichen, und zwar in. Abhängigkeit vom Körpergewicht, der Art des Applikationsweges, vom individuellen Verhalten gegenüber dem Medikament, von der Art der Formulierung und vom Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Weiterhin Gegenstand der vorliegenden Erfindung sind pharmazeutische Formulierungen enthaltend wenigstens eine erfindungsgemäße Verbindung der allgemeinen Formel (I) bzw. deren Salze, Solvate und/oder Hydrate.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Verwendung von Wasser als Lösungsmittel gegebenenfalls organische Lösungsmittel als Hilfslösungsmittel verwendet werden können. Hierzu geeignete Lösungsmittel, Trägerstoffe, Emulgier- und Dispergiermittel sind dem Fachmann bekannt.

Im Allgemeinen liegt die effektive Dosis abhängig vom Geschlecht, Alter und der körperlichen Verfassung des Patienten bei Erwachsenen bei einer Menge von 10 bis 1000 mg/Tag, bevorzugt bei 20 bis 500 mg/Tag, und zwar entweder in einer oder mehreren Einzelgaben

Trotzdem kann es erforderlich sein, von den zuvor genannten effektiven Dosen abzuweichen, und zwar in Abhängigkeit vom Körpergewicht, der Art des Applikationsweges, vom individuellen Verhalten gegenüber dem Medikament, von der Art der Formulierung und vom Zeitpunkt bzw. Intervall, zu welchem die Verabreichung erfolgt. So kann es in einigen Fällen ausreichend sein, mit weniger als der vorgenannten Mindestdosis auszukommen, während in anderen Fällen die genannte obere Grenze überschritten werden muss. Im Falle der Applikation größerer Dosen kann es empfehlenswert sein, diese in mehreren Einzelgaben über den Tag zu verteilen, und zwar entweder in mehreren Einzelgaben oder als Dauerinfusion.

Die erfindungsgemäßen Verbindungen der allgemeinen Formel (I) bzw. deren Salze, Solvate und/oder Hydrate zeigen insbesondere gute Löslichkeit, was für bestimmte Darreichungsformen von besonderem Vorteil sein kann.

Die im Folgenden aufgeführten Beispiele dienen der beispielhaften Erläuterung der Erfindung und sind nicht als Beschränkung aufzufassen.

### Beispiele

### Beispiel 1: Darstellung von 6-Methyl-2-[4-(3-oxo-morpholin-4-yl)-phenylamino]-nicotinsäure

4.30 g (25.0 mmol) 2-Chlor-6-methyl-nicotinsäure, 5.05 g (26.3 mmol) 4-(4-Amino-phenyl)-morpholin-3-on und 6 ml Pyridin wurden in 90 ml absolutiertem CHCl₃ unter Stickstoff-Schutzgasatmosphäre 9 Tage unter Rückfluss gerührt. Nach vollständigem Umsatz (Umsatzkontrolle mittel DC) wurde die Reaktionsmischung auf 20°C abgekühlt, der Rückstand abfiltriert und mit 30 ml CHCl₃ nachgewaschen. Zusätzlich wurde das Filtrat einrotiert, der ölige Rückstand in 45 ml CH₂Cl₂ gelöst und mit 20 ml CH₃OH versetzt. Anschließend wurde das CH₂Cl₂ im Vakuum entfernt, wobei nach Abkühlen der Lösung auf 0°C weiterer Feststoff isoliert werden konnte und zusammen mit dem Filterrückstand insgesamt 4.48 g (13,7 mmol; 55 %) 6-Methyl-2-[4-(3-oxo-morpholin-4-yl)-phenylamino]-nicotinsäure als gelber Feststoff erhalten wurden.
Schmelzpunkt: 242-244°C
1H-NMR (DMSO-d₆), ppm: δ 2.44 ((s, 3H), 3,72 (t, 2H), 3,96 (t, 2H), 4,19 (s, 2H), 6.75 (dd, 1H), 7,32 (dd, 2H), 7.78 (dd, 2H), 8.13 (dd, 1H), 10.48 (s, 1H), 13.26 (br s, 1H)

## Patentansprüche

1. Verbindung der allgemeinen Formel (I), 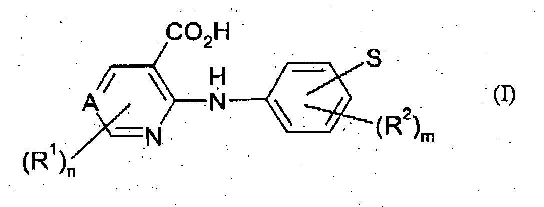 worin
A für CH, CR¹ oder N steht,
R¹ unabhängig voneinander für H, OH, C₁-C₄-Alkyl, teil- oder perfluoriertes C₁-C₄-Alkyl, C₁-C₁₄-Alkoxy, teil- oder perfluoriertes C₁-C₄-Alkoxy, Amino, Mono- oder Diamino-C₁-C₄-alkyl, Nitro, Carboxyl, C(S)NH₂, C(O)NH₂, Cyano oder Halogen steht,
n für 0, 1 oder 2 steht,
R² unabhängig voneinander für H, OH, C₁-C₄-Alkyl, teil- oder perfluoriertes C₁-C₄-Alkyl, C₁-C₄-Alkoxy, teil- oder perfluoriertes C₁-C₄-Alkoxy, Amino, Mono- oder Diamino-C₁-C₄-alkyl, Nitro, Carboxyl, C(S)NH₂, C(O)NH₂, Cyano oder Halogen steht,
m für 0, 1, 2, 3 oder 4 steht,
S für einen Rest ausgewählt aus den allgemeinen Formeln (II-a) bis (II-d) 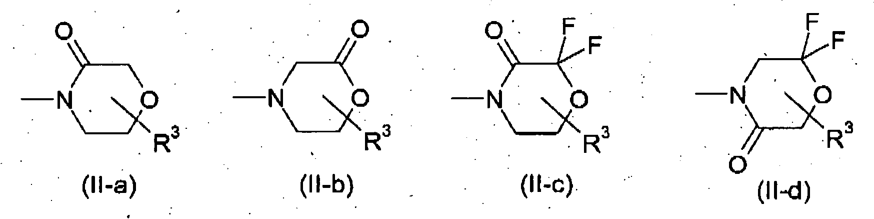 steht, worin
R³ für H, C₁-C₄-Alkyl, gegebenenfalls substituiertes C₄-C₂₄-Aryl, in dem keines, eines, zwei oder drei Gerüstkohlenstoffatome pro Cyclus durch Heteroatome, im gesamten Molekül mindestens jedoch ein Gerüstkohlenstom durch ein Heteroatom, ausgewählt aus der Gruppe
Stickstoff, Schwefel oder Sauerstoff ersetzt sein können, oder gegebenenfalls substituiertes C₅-C₁₈-Aralkyl steht,
oder Salze der Verbindungen der allgemeinen Formel (I) oder Solvate oder Hydrate der Verbindungen der allgemeinen Formel (I) oder deren Salzen.

2. Verbindung gemäß Anspruch 1, **dadurch gekennzeichnet, dass** R¹ für H, F, OH, Methyl, Mcthoxy, Trifluormethyl oder Difluormethyl steht.

3. Verbindung gemäß Anspruch 1 oder 2, **dadurch gekennzeichnet, dass** R² für H, F, Cl, CN, OH, Methyl, Methoxy, Trifluormethyl, Difluormethyl, C(S)NH₂ oder C(O)NH₂ steht.

4. Verbindung gemäß wenigstens einem der Ansprüche 1 bis 3, **dadurch gekennzeichet, dass**
R¹, R² unabhängig voneinander für H, F oder Methyl stehen und
S für einen Rest der allgemeinen Formel (II-a) steht, 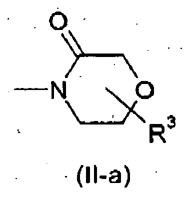 worin
R³ für H oder Methyl steht.

5. Verbindung gemäß wenigstens einem der Ansprüche 1 bis 4, **dadurch gekennzeichnet, dass** es sich dabei um eine Verbindung ausgewählt aus den Formeln (I-a) bis (I-c) handelt

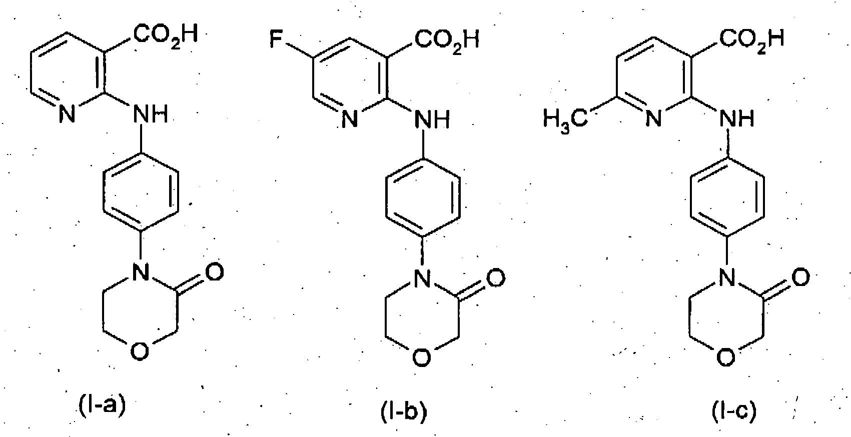

6. Verfahren zur Herstellung von Verbindungen gemäß wenigstens einem der Ansprüche 1 bis 5, **dadurch gekennzeichnet, dass** Verbindungen der allgemeinen Formel (III), 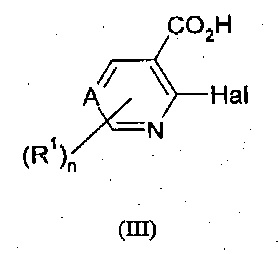 worin
Hal für Cl, Br oder I steht und
A und R¹ die in wenigstens einem der Ansprüche 1 bis 6 angegebene Bedeutung haben,
mit Verbindungen der allgemeinen Formel (IV) 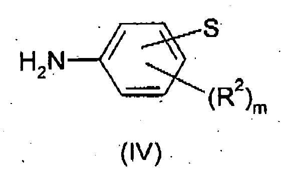 worin R² und S die in wenigstens einem der Ansprüche 1 bis 5 angegebene Bedeutung haben,
umgesetzt werden.

7. Verfahren gemäß Anspruch 6, **dadurch gekennzeichnet, dass** Hal für Cl steht.

8. Verbindung der allgemeinen Formel (IV), 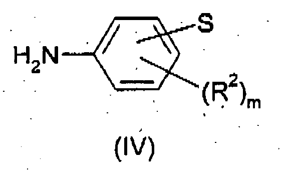 **dadurch gekennzeichnet, dass** R² die in wenigstens einem der Ansprüche 1 bis 5 angegebene Bedeutung hat und S für einen Rest ausgewählt aus den allgemeinen Formeln (II-b) bis (II-d) 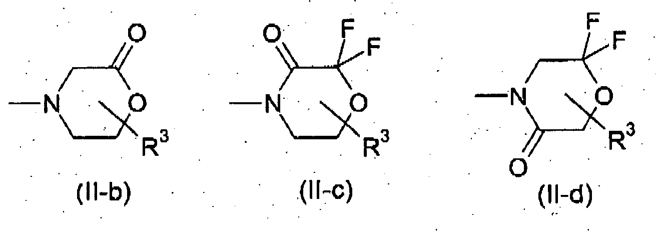 steht,
worin R³ die in wenigstens einem der Ansprüche 1 bis 5 angegebene Bedeutung hat.

9. Verbidung gemäß Anspruch 8, **dadurch gekennzeichnet, dass** es sich um die Verbindung der Formel (IV-c) oder (IV-d) handelt

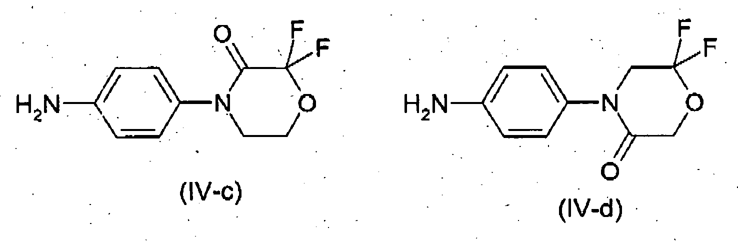

10. Verwendung der Verbindungen gemäß Anspruch 8 oder 9 zur Herstellung pharmazeutischer Wirkstoffe gemäß Anspruch 1.

11. Verwendung der Verbindungen gemäß einem der Anspruche 1 bis 5 zur Herstellung eines Medikaments zur Behandlung von Hepatitis C.

12. Pharmazeutische Formulierung enthaltend wenigstens eine Verbindung gemäß wenigstens einem der Ansprüche 1 bis 5.

## Claims

1. Compound of the general formula (I), 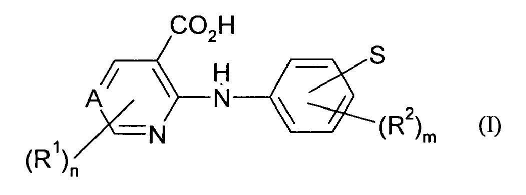 in which
A represents CH, CR¹ or N,
R¹ independently of one another represent H, OH, C₁-C₄-alkyl, partially fluorinated or perfluorinated C₁-C₄-alkyl, C₁-C₄-alkoxy, partially fluorinated or perfluorinated C₁-C₄-alkoxy, amino, mono- or diamino-C₁-C₄-alkyl, nitro, carboxyl, C(S)NH₂, C(O)NH₂, cyano or halogen,
n represents 0, 1 or 2,
R² independently of one another represent H, OH, C₁-C₄-alkyl, partially fluorinated or perfluorinated C₁-C₄-alkyl, C₁-C₄-alkoxy, partially fluorinated or perfluorinated C₁-C₄-alkoxy, amino, mono- or diamino-C₁-C₄-alkyl, nitro, carboxyl, C(S)NH₂, C(O)NH₂, cyano or halogen,
m represents 0, 1, 2, 3 or 4,
S represents a radical selected from the general formulae (II-a) to (II-d) 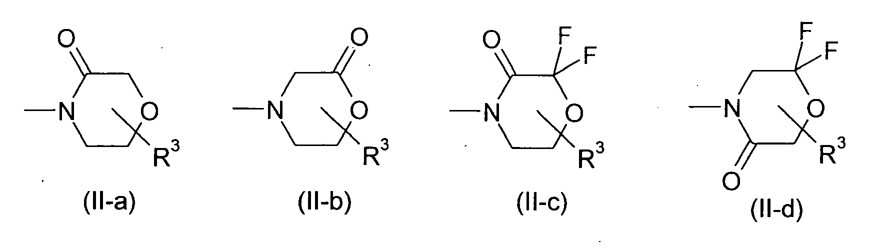 in which
R³ represents H, C₁-C₄-alkyl, optionally substituted C₄-C₂₄-aryl in which no, one, two or three carbon atoms in the skeleton of each ring, but at least one carbon atom in the skeleton of the entire molecule, may be replaced by (a) heteroatom(s) selected from the group consisting of nitrogen, sulphur and oxygen, or optionally substituted C₅-C₁₈-aralkyl,
or salts of the compounds of the general formula (I) or solvates or hydrates of the compounds of the general formula (I) or their salts.

2. Compound according to Claim 1, **characterized in that** R¹ represents H, F, OH, methyl, methoxy, trifluoromethyl or difluoromethyl.

3. Compound according to Claim 1 or 2, **characterized in that** R² represents H, F, Cl, CN, OH, methyl, methoxy, trifluoromethyl, difluoromethyl, C(S)NH₂ or C(O)NH₂.

4. Compound according to at least one of Claims 1 to 3, **characterized in that**
R¹, R² independently of one another represent H, F or methyl and
S represents a radical of the general formula (II-a), 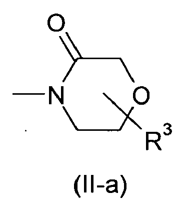 in which
R³ represents H or methyl.

5. Compound according to at least one of Claims 1 to 4, **characterized in that** it is a compound selected from the formulae (I-a) to (I-c)

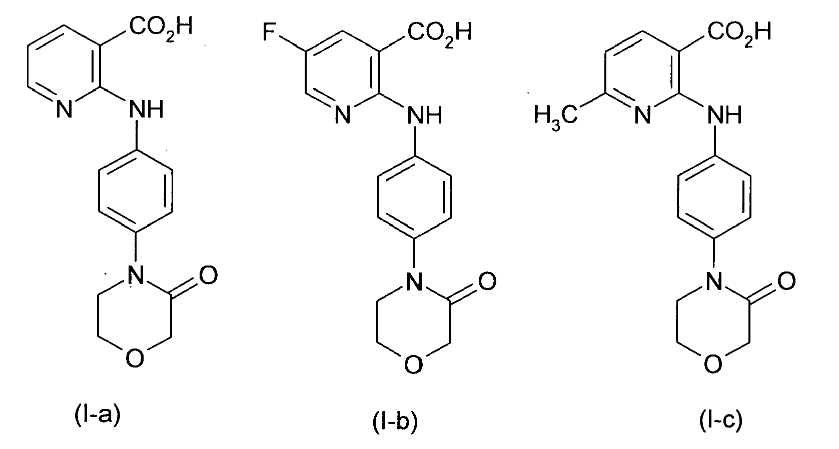

6. Process for preparing compounds according to at least one of Claims 1 to 5, **characterized in that** compounds of the general formula (III), 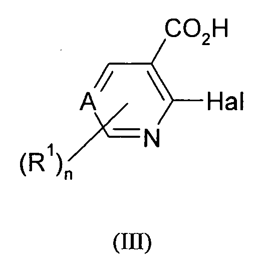 in which
Hal represents Cl, Br or I and
A and R¹ have the meaning given in at least one of Claims 1 to 6
are reacted with compounds of the general formula (IV) 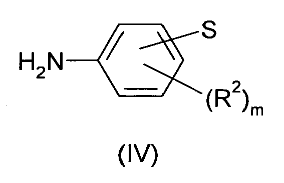 in which R² and S have the meaning given in at least one of Claims 1 to 5.

7. Process according to Claim 6, **characterized in that** Hal represents Cl.

8. Compound of the general formula (IV), 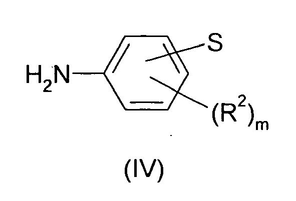 **characterized in that** R² has the meaning given in at least one of Claims 1 to 5 and S represents a radical selected from the general formulae (II-b) to (II-d) 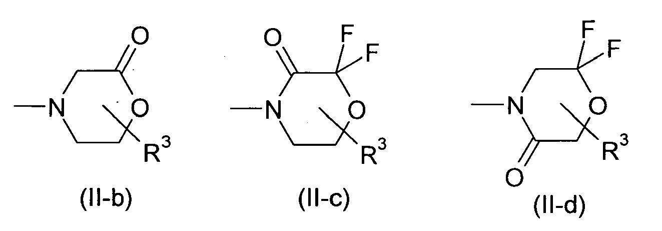 in which R³ has the meaning given in at least one of Claims 1 to 5.

9. Compound according to Claim 8, **characterized in that** it is the compound of the formula (IV-c) or (IV-d)

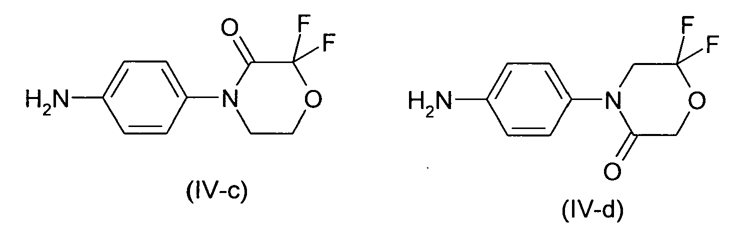

10. Use of the compounds according to Claim 8 or 9 for preparing pharmaceutically active compounds according to Claim 1.

11. Use of the compounds according to one of Claims 1 to 5 for preparing a medicament for treating hepatitis C.

12. Pharmaceutical formulation, comprising at least one compound according to at least one of Claims 1 to 5.

## Revendications

1. Composé de formule générale (I) 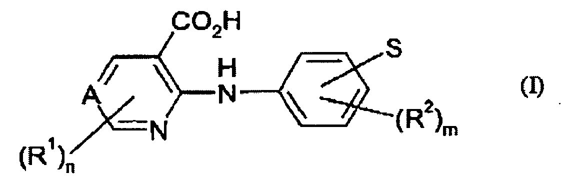 où
A représente CH, CR¹ ou N,
R¹ représente, indépendamment l'un de l'autre, H, OH, C₁-C₄-alkyle, C₁-C₄-alkyle partiellement fluoré ou perfluoré, C₁-C₄-alcoxy, C₁-C₄-alcoxy partiellement fluoré ou perfluoré, amino, monoamino-C₁-C₄-alkyle ou diamino-C₁-C₄-alkyle, nitro, carboxyle, C(S)NH₂, C(O)NH₂, cyano ou halogène,
n vaut 0, 1 ou 2,
R² représente, indépendamment l'un de l'autre, H, OH, C₁-C₄-alkyle, C₁-C₄-alkyle partiellement fluoré ou perfluoré, C₁-C₄-alcoxy, C₁-C₄-alcoxy partiellement fluoré ou perfluoré, amino, monoamino-C₁-C₄-alkyle ou diamino-C₁-C₄-alkyle, nitro, carboxyle, C(S)NH₂, C(O)NH₂, cyano ou halogène,
m vaut 0, 1, 2, 3 ou 4,
s représente un radical choisi parmi les formules générales (II-a) à (II-d) 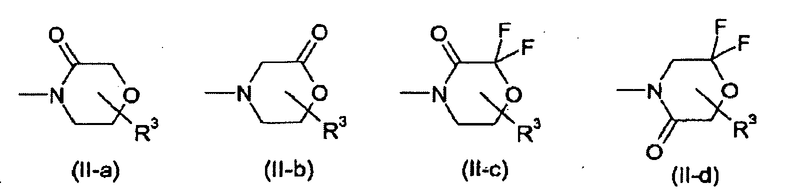 dans laquelle
R³ représente H, C₁-C₄-alkyle, C₄-C₂₄-aryle le cas échéant substitué, dans lequel aucun, un, deux ou trois atomes de carbone de la structure par cycle peuvent être remplacés par des hétéroatomes, dans la molécule globale cependant au moins un carbone de la structure peut être remplacé par un hétéroatome, choisi dans le groupe formé par l'azote, le soufre ou l'oxygène, ou C₅-C₁₈-aralkyle le cas échéant substitué,
ou des sels des composés de formule générale (I) ou des solvates ou des hydrates des composés de formule générale (I) ou de leurs sels.

2. Composé selon la revendication 1, **caractérisé en ce que** R¹ représente H, F, OH, méthyle, méthoxy, trifluorométhyle ou difluorométhyle.

3. Composé selon la revendication 1 ou 2, **caractérisé en ce que** R² représente H, F, Cl, CN, OH, méthyle, méthoxy, trifluorométhyle, difluorométhyle, C(S)NH₂ ou C(O)NH₂.

4. Composé selon au moins l'une quelconque des revendications 1 à 3, **caractérisé en ce que**
R¹, R² représentent, indépendamment l'un de l'autre, H, F ou méthyle et
S représente un radical de formule générale (II-a) 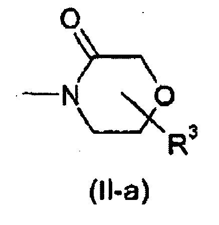
où R³ représente H ou méthyle.

5. Composé selon au moins l'une quelconque des revendications 1 à 4, **caractérisé en ce qu'**il s'agit d'un composé choisi parmi les formules (I-a) à (I-c)

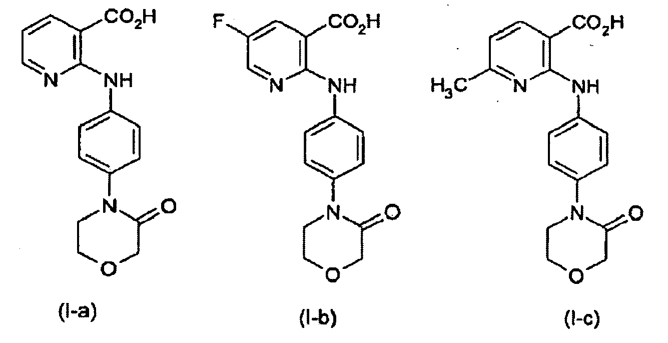

6. Procédé pour la préparation de composés selon au moins l'une quelconque des revendications 1 à 5, **caractérisé en ce qu'**on transforme des composés de formule générale (III) 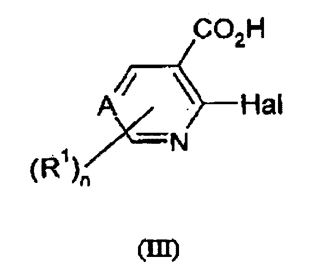 où
Hal représente Cl, Br ou I et
A et R¹ présentent la signification indiquée dans au moins l'une quelconque des revendications 1 à 6, avec des composés de formule générale (IV) 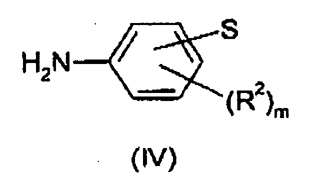
dans laquelle R² et S présentent la signification indiquée dans au moins l'une quelconque des revendications 1 à 5.

7. Procédé selon la revendication 6, **caractérisé en ce que** Hal représente Cl.

8. Composé de formule générale (IV) 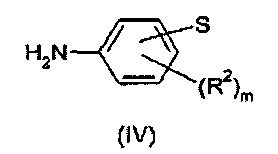 **caractérisé en ce que** R² présente la signification indiquée dans au moins l'une quelconque des revendications 1 à 5 et S représente un radical choisi parmi les formules générales (II-b) à (II-d) 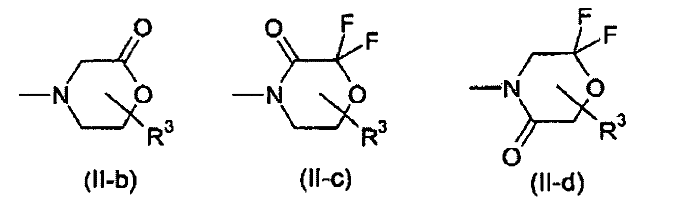 dans lesquelles R³ présente la signification indiquée dans au moins l'une quelconque des revendications 1 à 5.

9. Composé selon la revendication 8, **caractérisé en ce qu'**il s'agit du composé de formule (IV-c) ou (IV-d)

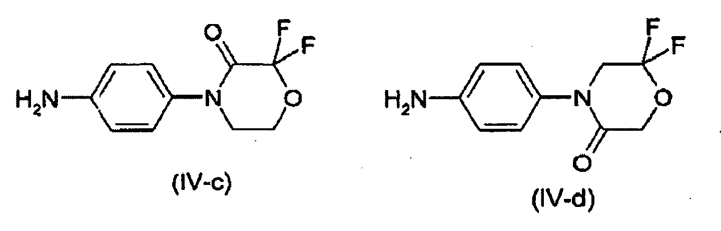

10. Utilisation des composés selon la revendication 8 ou 9 pour la préparation de substances actives pharmaceutiques selon la revendication 1.

11. Utilisation des composés selon l'une quelconque des revendications 1 à 5 pour la préparation d'un médicament destiné au traitement de l'hépatite C.

12. Formulation pharmaceutique contenant au moins un composé selon au moins l'une quelconque des revendications 1 à 5.
